Europäisches Patentamt

European Patent Office

Office européen des brevets

(19)

(11) Publication number: **0 128 849 B1**

(12)

# EUROPEAN PATENT SPECIFICATION

(45) Date of publication of patent specification: **30.09.92**

(51) Int. Cl.⁵: **A61K 35/14**, A61K 37/02, A61K 35/50

(21) Application number: **84450016.5**

(22) Date of filing: **04.06.84**

(54) **Purified transforming growth factor-beta derived from human platelets and placentas.**

(30) Priority: **03.06.83 US 500832**
**03.06.83 US 500927**

(43) Date of publication of application:
**19.12.84 Bulletin 84/51**

(45) Publication of the grant of the patent:
**30.09.92 Bulletin 92/40**

(84) Designated Contracting States:
**AT BE CH DE FR GB IT LI NL SE**

(56) References cited:
**WO-A-84/01106**

**BIOLOGICAL ABSTRACTS, vol. 77, 1984, abstract no. 440; R.K. ASSONIAN et al.: "Transforming growth factor-beta in human platelets: Identification of a major storage site, purification and characterization" & J. BIOL. CHEM. 258(11), 7155-7160, 1983**

**PROC. NATL. ACAD. SCI. USA, vol. 80, June 1983, pages 3676-3680; C.A. FROLIK et al.: "Purification and initial characterization of a type beta transforming growth factor from human placenta"**

(73) Proprietor: **THE UNITED STATES OF AMERICA as represented by the Secretary, United States Department of Commerce National Technical Information Service, Office of Government Inventions and Patents, 5285 Port Royal Road Springfield, Virginia 22161(US)**

(72) Inventor: **Frolik, Charles A.**
**14 Brighton Terrace**
**Gaithersburg Maryland 20877(US)**
Inventor: **Assoian, Richard K.**
**8809 Bradford Road**
**Silver Spring Maryland 20901(US)**
Inventor: **Sporn, Michael B.**
**6630 Marywood Road**
**Bethesda Maryland 20817(US)**
Inventor: **Roberts, Anita B.**
**9919 Harrogate Road**
**Bethesda Maryland 20817(US)**

(74) Representative: **Strehl, Schübel-Hopf, Groening**
**Maximilianstrasse 54 Postfach 22 14 55**
**W-8000 München 22(DE)**

EP 0 128 849 B1

BIOLOGICAL ABSTRACTS/RRM, vol. 25, 1983, ref. no. 51811; R.K. ASSOIAN et al.: "Platelet derived transforming growth factor beta" & FEDERATION PROCEEDINGS (USA) 1983, vol. 42, no. 7, abstract 428

BIOLOGICAL ABSTRACTS/RRM, vol. 25, 1983, ref. no. 45382; C.A. FROLIK et al.: "Purification and initial characterization of a human placental type beta transforming growth factor" & FEDERATION PROCEED-INGS (USA) 1983, vol. 42, no. 7, abstract no. 440

**Description**

This invention relates to the isolation and purification of beta-type transforming growth factor (TGF-beta) from human blood platelets and human placental tissue, respectively.

Transforming growth factors are biologically active peptides which induce anchorage-dependent, non-neoplastic cells to lose contact inhibition and to undergo anchorage-independent growth. This property is unique to transforming growth factors (TGFs), and it can be quantitated by measuring the formation of colonies of cells in soft agar. Two classes of transforming growth factors have been identified on the basis of their relationship with epidermal growth factor (EGF). Type alpha TGFs are defined by an ability to compete with $125_I$-labeled EGF for binding to the EGF cell surface receptor. They are single chain, low molecular weight ($M_r < 10,000$) peptides which have been found in the conditioned medium of transformed cell lines.

In contrast, type beta TGFs do not bind to the EGF receptor, but they require EGF or TGF-$\alpha$ for expression of their biological activity. [See Roberts, et al., Cold Spring Harbor Conf. Cell Proliferation 9:319-332 (1982); Anzano, et al., Anal. Biochem. 125:217-224 (1982); and Anzano, et al., Cancer Res. 42:4776-4778 (1982).] Although type beta TGFs are present in transformed cells, they have also been detected in a variety of non-neoplastic tissues [see Roberts, et al., Proc. Nat. Acad. Sci. U.S.A. 78:5339-5343 (1981)] suggesting a physiological role for these factors in modulating normal cell growth. One likely role for such growth factors is to participate in tissue repair and regeneration; TGF-beta, in conjunction with EGF, is an effective promotor of wound healing in vivo. The recent identification of TGF activitiy in serum and platelets further suggests a possible role for TGF-beta in tissue repair [see Childs, et al., Proc. Nat. Acad. Sci. U.S.A. 79:5312-5316 (1982)].

The biological effect of TGF-beta has been well documented, but little is known about its major site(s) of synthesis and storage. Moreover, the lack of purified TGF-beta has precluded many studies of its structure and mechanism of action.

SUMMARY OF THE INVENTION

Subject matter of the present invention is a beta-type transforming growth factor derivable from human blood platelets or human placentas, in the form of a homogeneous protein causing half-maximal growth stimulation of NRK-fibroblast indicator cells in soft agar at a protein concentration of less than $5 \times 10^{-12}$ M, wherein said cells comprise colonies larger than 3000 $\mu m^2$ and wherein said growth stimulation is increased approximately 1000-fold by the presence of EGF or TGF-$\alpha$.

It has been discovered that platelets contain 40-100 fold more TGF-beta than do the other non-neoplastic tissues examined to date. Complete purification of this platelet factor now shows that TGF-beta is a polypeptide of about 25,000 daltons which is probably composed of two 12,500-dalton subunits held together by disulfide bonds. Its molecular weight, subunit structure, and amino acid composition differ from those of platelet-derived growth factor (PDGF). In contrast to most growth factors, platelet-derived TGF-beta does not appear to exert its growth promoting property by directly stimulating total DNA synthesis.

Acidic ethanol extracts of human platelets induced non-neoplastic NRK-fibroblasts to undergo anchorage-independent growth. Less than 100 ng/ml of the crude extract elicits 50% of the maximal biological response when assayed in the presence of epidermal growth factor (2.5 ng/ml). In the absence of epidermal growth factor the potency of the extract decreased 1000-fold. These results show that platelets contain a type beta transforming growth factor (TGF-beta). The specific activity of the platelet extract is 100-fold greater than that of other non-neoplastic tissues. The growth factor was purified to homogeneity by sequential gel filtration, first in the absence and then in the presence of urea. These results, as well as its amino acid composition and its lack of strong mitogenic activity, show that this protein is distinct from platelet-derived growth factor. When completely purified, platelet-derived TGF-beta elicits 50% of its maximal biological response at concentrations less than $5 \times 10^{-12}$ M.

It has also been discovered that a polypeptide transforming growth factor (TGF), which induces anchorage-dependent rat kidney fibroblasts to grow in soft agar, can be isolated from human placenta and purified to homogeneity. This polypeptide is classified as a type beta TGF because it does not compete with epidermal growth factor (EGF) for membrane receptor sites but does require EGF for induction of anchorage-independent growth of indicator cells.

Purification of this peptide was achieved by acid-ethanol extraction of the placenta, followed by gel filtration, cation-exchange and high-pressure liquid chromatography of the acid soluble proteins. Homogeneity of the TGF-beta from the final column was shown by its constant specific activity and amino acid composition across the peak of soft agar colony forming activity and by its migration as a single band at 23,000 to 25,000 molecular weight after $NaDodSO_4$-polyacrylamide gel electrophoresis. Under reducing conditions, the protein migrated on a gel as a single band at 13,000 molecular weight. The purified placental TGF-beta caused half maximal growth stimulation of indicator cells in soft agar at 64-72 pg/ml ($3 \times 10^{-12}$ M) in the presence of 2 ng/ml ($3.4 \times 10^{-10}$ M) of EGF.

Although TGFs were originally found in tumor cells and were postulated to be involved in transformation and neoplastic cell growth, their presence in adult cells and tissues, in platelets, and in embryos [as reported in Twardzik, et al., Cancer Res. 42:590-593 (1982)] imply that TGFs have a normal physiological function as well. The purification of placental and platelet-derived TGF-beta to homogeneity, in accordance with the present invention, facilitates investigation of this function, since it permits the development of both receptor binding and radioimmunioassays. Such assays not only allow a specific, quick procedure for quantitation of TGF-beta but will also permit investigation of the mechanism of action and the control of expression of TGF-betas under normal and neoplastic conditions. Moreover, structural analysis of purified TGF-beta provides information for initiation of cloning experiments. This information will allow eventual production of large quantities of human TGF-beta, which is expected to have useful therapeutic applications in enhancement of wound healing and tissue repair.

DETAILED DESCRIPTION OF THE INVENTION

1. TGF-Beta Derived from Platelets

Transforming growth factors have been detected in a variety of non-neoplastic tissues, but major sites of storage have not been identified. However, a comparison of both specific activities in initial extracts and yields of purified TGF-beta shows that platelets are a major storage site for the growth factor; they contain 40-100 fold more TGF-beta than do the other non-neoplastic tissues which have been examined. This finding, in conjunction with the known role of platelets in wound healing, supports the hypothesis that at least one physiological role of TGF-beta is to facilitate tissue repair and regeneration.

The total purification of platelet-derived TGF-beta was facilitated by both the high specific activity of the platelet extract and the aberrant elution of the polypeptide during gel filtration. Contaminants with molecular weights similar to the TGF (25,000 daltons) were removed on a column of acrylamide in 1 M acetic acid. In this system TGF-beta elutes with proteins of half its mass. (An apparent discrepancy in one fraction--high biological activity and no detectable protein at 25,000 daltons--was due to the fact that detection of TGF-beta by bioassay is at least 100-fold more sensitive than chemical detection of the protein by electrophoresis and silver staining.) Addition of urea to the eluant prevented this retardation and resulted in the complete separation of TGF-beta from the lower molecular weight peptides.

The overall recovery of biological activity from the purification procedure is somewhat low (about 5%), but control studies showed that other platelet factors modulate TGF-beta action. (The specific activity of TGF-beta decreased at least 10-fold when it was assayed in medium containing 10% plasma-derived rather than whole-blood derived serum; data not shown.) Removal of these factors during the purification procedure may well explain the observed decreases in total TGF-beta biological activity. The maximal biological activity of PDGF also requires the presence of other bio-active peptides.

Purified, platelet-derived TGF-beta was characterized chemically and biologically (Tables 1 and 2). Its molecular weight (25,000 daltons), subunit structure (two 12,500-dalton polypeptides indistinguishable by SDS-polyacrylamide gel electrophoresis), and amino acid composition differ from that of PDGF. Moreover, PDGF is a potent mitogen whereas platelet-derived TGF-beta is, at best, weakly mitogenic. Using similar biochemical criteria platelet-derived TGF-beta is also distinct from the platelet protein family comprised of CTAP-III, beta-thromboglobulin, and platelet factor 4.

The role of platelets as a source of growth factors has received widespread attention since the identification of PDGF. A platelet-derived peptide (C-TAP III; 9300 daltons) has been purified to homogeneity and shown to be mitogenic for connective tissue cells. Two platelet growth factors distinct from PDGF have been identified on the basis of their isoelectric points. Recently, it has been shown that TGF activity is present in platelets and that the activity is enhanced by EGF. These studies with partially purified preparations yielded two active components during gel filtration ($M_r$ = 12-16,000 and 6,000). The larger protein is likely the 25,000 dalton TGF-beta described herein eluting with an aberrantly low molecular weight during gel filtration in the absence of denaturant. The smaller TGF was not detected but attention has been focused only on the most active TGF species in platelets. Transforming growth factors having specific activities less than 10% of that of the 25,000-dalton TGF-beta would not be detected with the activity limits imposed herein.

Studies implicating PDGF in atherosclerosis and control of cell division have emphasized its physiological release from platelets during their aggregation at a site of injury. However, the characterization of PDGF as a competence factor suggests that platelet-mediated control of cell growth likely involves a complex synergism between several bio-active peptides. Platelet-derived TGF-beta has strong growth promoting ability, but it is not a strong mitogen. This unusual combination of biological properties suggests that this protein may play a unique role in those physiological and pathological processes where platelet-derived factors modulate cell proliferation.

(A) Examples of Platelet-Derived TGF-Beta Purification and Analysis

Platelet Extraction: Platelet concentrates (20-30 units, 2-5 days old) were obtained through the courtesy of the National Institutes of Health Blood Bank (Bethesda, Maryland, U.S.A.) and centrifuged (3200 x g, 30 min, ) to remove remaining plasma proteins. The platelets were washed twice by suspension in 500-ml portions of Tris-HCl/citrate buffer, pH 7.5 and centrifugation as described above. Washed platelets (20-30 g wet weights) were added to a solution of acidic ethanol prepared as described elsewhere and immediately extracted in a homogenizer (4 ml acidic ethanol per g platelets). After incubation overnight at 4° C, precipitated proteins were removed by centrifugation, and the resulting supernatant was adjusted to pH 3 by addition of $NH_4OH$. Proteins and TGF activity were precipitated from the solution (overnight at 4°C) by addition of ethanol (2 vol, 0°C) and ethyl ether (4 vol, 0°C). The precipitate was collected by centrifugation and suspended in 1 M acetic acid (10 ml). TGF activity was solubilized during an overnight extraction at 4°C. Centrifugation clarified the solution; the supernatant was freeze-dried or subjected directly to gel filtration. The amount of protein in the extract was determined by weight or by reaction with Coomassie Blue using bovine plasma albumin as reference.

Purification of Platelet-Derived TGF-Beta: The solubilized platelet extract (10 ml in 1 M acetic acid) was gel-filtered at a flow rate of 20 ml/h on a column (4.4 x 115 cm) of acrylamide gel equilibrated in 1 M acetic acid. Fractions containing 5 ml were collected. The elution position of TGF-beta was determined by bioassay as described below, and the fractions containing the peak of activity were pooled and freeze-dried. The amount of protein in the pool was determined as described above. The residue was dissolved in 0.5 ml of 1 M acetic acid containing 8 M ultra-pure urea and gel-filtered at a flow rate of 3 ml/h on a column (1.6 x 85 cm) of acrylamide which had been equilibrated in the sample solvent. Fractions containing 0.5 ml were collected. (To preclude the formation of cyanate in the solvent, the ultra-pure urea was dissolved at pH 2 in 1 M acetic acid. The resulting solution was adjusted to final conditions by addition of glacial acetic acid and water.) Aliquots of selected column fractions were tested for TGF-beta activity. Fractions containing the peak of TGF-beta activity were pooled, dialyzed against 1 M acetic acid to remove urea, and quick-frozen for storage at -20°C. The amount of TGF-beta in the final solution was determined by amino acid analysis (see below).

Bioassay of TGF-Beta: The bioassay of TGF-beta determines the ability of the polypeptide to induce anchorage-independent growth in non-neoplastic NRK-fibroblasts by measuring the formation of colonies of cells in soft agar. The assay was performed as described in Roberts, et al., Proc. Nat. Acad. Sci. U.S.A. 77:3494-3498 (1980) except that 1) 3500 cells were used per dish, 2) incubation proceeded for 7 days at 37°C in a humidified atmosphere of 10% $CO_2$ in air, and 3) TGF-beta activity was determined in the presence of EGF (2.5 ng/ml). Samples were sterilized in 1 M acetic acid and freeze-dried in the presence of bovine serum albumin (100 $\mu$g) as carrier prior to analysis. Stained colonies were quantitated by number and size. One unit of TGF-beta activity is defined as that biological response resulting in 50% of maximal colony formation (colony size > 3000 $\mu m^2$) in the presence of Epidermal Growth Factor (EGF) (2.5 ng/ml). The maximal response of the assay is about 2500 colonies ( >3000 $\mu m^2$) per dish.

Mitogen Assay: NRK-fibroblasts were suspended in medium (Dulbecco's Modified Eagles Medium supplemented with 100 units per ml penicillin and 100 $\mu$g per ml streptomycin), 10% in calf serum. Cells (4 x $10^3$ in 0.1 ml) were seeded in 96-well microtitre plates and incubated overnight. (All incubations proceeded at 37 C in a humidified atmosphere of 10% $CO_2$ in air.) The resulting monolayers were washed twice with 0.2-ml portions of serum-free medium and once with 0.2 ml of medium containing 0.2% calf serum. DME, 0.2% in calf serum (100 $\mu$l), was added to the washed monolayers. The cells were incubated for 3-4 days during which time they reached about 75% confluency. Test samples (50 $\mu$l, freeze-dried from 1 M acetic acid and redissolved in 20 $\mu$l of sterile 4 mM HCl and 40 $\mu$l of serum-free medium) were added to the growth arrested cells. After incubation (17 h), $^3$H-thymidine (80 Ci/mmol) was added (1 $\mu$Ci in 50 $\mu$l of serum-free medium). Four hours later the medium was removed, and the cells were fixed (10 min at 4°C) with ice-cold 5% trichloroacetic acid (0.2 ml). Fixed cells were washed 4 times with 0.2-ml portions of 5% trichloroacetic acid. Precipitated radioactivity was solubilized by incubation in 0.5 M NaOH (0.15 ml per well for 30 min at 37° C).

Amino Acid Analysis: The amino acid composition of purified TGF-beta (5-20 pmol) was determined by hydrolysis (24 h at 110°C) in vacuo with 6 N HCl containing a trace amount of phenol. Analysis was performed on a modified amino acid analyzer using o-phthalaldehyde reagent, a fluorometer, and the following sodium citrate buffers: pH 3.28 (adjusted to pH 3.25 by addition of 300 $\mu$l of 6 N HCl and 18 ml isopropanol per 900 ml buffer), pH 4.25, and pH 7.4. To remove trace amounts of free amino acids from preparations of TGF-beta, the peptide solution (10-50 pmol in 0.1-0.5 ml of 1 M acetic acid) was dialyzed in a microdialysis unit against 1 M acetic acid prepared with HPLC-grade water. Contaminating amino acids remaining after dialysis were quantitated by analyzing equivalent amounts of TGF-beta with and without hydrolysis. The amount of each amino acid in the unhydrolyzed sample was about 5% of that in the hydrolyzed sample except for serine (15%) and glycine (20%).

(B) Functional Characteristics of Platelet-Derived TGF-Beta

The biological properties of purified, platelet-derived TGF-beta are shown below in Table 1. In the presence of EGF, the TGF elicits near maximal transforming activity at a concentration of 1 ng/ml. In agreement with the data of others using impure TGF-beta, the activity of the growth factor is destroyed by reduction; stimulation of colony formation by an EGF/reduced TGF-beta mixture was no greater than that of EGF alone. Moreover, TGF-beta, assayed in the absence of EGF, gave the basal level (shown by 10% calf serum) of transforming activity. Other experiments showed that TGF-beta (1 ng/ml) does not compete for the binding of $^{125}$I-labeled EGF to the EGF receptor.

TGF-beta can be detected in the platelet extract at protein concentrations showing no mitogenic activity. Table 1 shows that purified TGF-beta (1 ng/ml) does not stimulate $^3$H-thymidine incorporation into NRK-fibroblasts despite the fact that these cells respond to established mitogens. [Decreased $^3$H-thymidine incorporation, relative to basal was observed with TGF-beta when used at concentrations greater than 0.1 ng/ml. At no concentration tested (0.01-10 ng/ml) did the TGF stimulate $^3$H-thymidine incorporation.] In addition to confirming that platelet-derived TGF-beta is biologically distinct from PDGF, these data suggest that the role of TGF-beta in inducing cell growth in soft agar may be unrelated to a direct stimulation of total DNA synthesis.

The sensitivity of TGF-beta to treatment with dithiothreitol (Table 1) indicates that disulfide bonds likely play an important role in conferring structure to the molecule. The molecular weight of platelet-derived TGF-beta, as determined by SDS-polyacrylamide gel electrophoresis, is affected by treatment with reductant. This result indicates that the native protein ($M_r$ = 25,000) is composed of two polypeptide chains of very similar molecular weight ($M_r$ = 12,500) which are maintained in covalent association by disulfide bonds. (The inability to detect contaminants in the presence as well as absence of reductant further confirms the purity of the protein.)

Table 2 shows the amino acid composition of purified, platelet-derived TGF-beta obtained after hydrolysis of the polypeptide in acid. As expected from the data of Table 1, half-cystine is present. The analysis differs from that of PDGF in the number of Thr, Glu, Val, Ile, Tyr, and Arg residues present per mol protein. Importantly, TGF-beta has about 10 more tyrosine residues per mol protein than does PDGF, indicating that the TGF does not arise from proteolysis of PDGF.

Table 1

| Biological Effects of Purified Platelet-Derived TGF-Beta | | |
|---|---|---|
| Sample | Number of Colonies (>3000 $\mu$m$^2$) | Amount of $^3$H-Thymidine Incorporation (CPM) |
| TGF-beta and EGF | 1980 | ND |
| reduced TGF-beta and EGF | 380 | ND |
| EGF | 400 | 45,200 |
| TGF-beta | 25 | 4,800 |
| calf serum (10%) | 12 | 86,000 |

This table shows the biological properties of purified TGF-beta at a concentration of 1 ng/ml (a concentration 10-fold greater than that yielding 50% of maximal transforming activity). EGF was used at 2.5 ng/ml, its concentration in the TGF-beta bioassay. The growth factors were dissolved in 1 M acetic acid with 10 $\mu$g BSA as carrier and freeze-dried prior to analysis. To prepare reduced TGF-beta, the lyophilized growth factor and BSA carrier were treated with a molar excess of dithiothreitol (0.05 M in 0.2 ml of 0.1 M sodium phosphate buffer, pH 7.4; 3 h at 37°C). The solution of reduced TGF-beta was acidified with acetic acid (40 μl), dialyzed against 1 M acetic acid in a microdialysis unit, and freeze-dried prior to analysis. EGF was added to the sample after dialysis. A mock reduction (performed in the absence of dithiothreitol) had no effect on TGF-beta transforming activity. In the mitogen assay, the basal level of $^3$H-thymidine incorporation (determined in the absence of mitogen) was 9000-10,000 CPM. The mitogenic activity of TGF-beta was not determined (ND) in the presence of EGF.

Table 2

| Amino Acid Composition of Platelet-Derived TGF-Beta | |
| --- | --- |
| amino acid | residues per mol (mean + range) |
| Aspartic Acid | 23 ± 2 |
| Threonine | 9 ± 1 |
| Serine | 17 ± 1 |
| Glutamic Acid | 26 ± 1 |
| Glycine | 21 ± 5 |
| Alanine | 17 ± 1 |
| Half-cystine | 16 ± 2 |
| Valine | 14 ± 1 |
| Methionine | 2 ± 0 |
| Isoleucine | 10 ± 1 |
| Leucine | 23 ± 2 |
| Tyrosine | 14 ± 1 |
| Phenylalanine | 6 ± 1 |
| Histidine | 6 ± 1 |
| Lysine | 18 ± 2 |
| Arginine | 10 ± 1 |
| (Proline) | ND |
| (Tryptophan) | ND |

This table shows the amino acid composition of purified, platelet-derived TGF-beta obtained after 24 h hydrolysis in 6 N HCl. The table compiles the results of three determinations. Half-cystine and methionine content were determined after hydrolysis of the performic acid-oxidized protein (100 μl reagent and 5-20 pmol TGF-beta). Proline (not detectable with o-phthalaldehyde) and tryptophan content were not determined (ND). The number of residues per mol for each amino acid was determined by "best fit" to a molecular weight of 25,000.

Table 3.   Partial Amino Acid Sequence of Platelet-Derived TGF-Beta

$$5 \qquad X*$$
Ala-Leu-Asp-Thr-Asn-Tyr-CMC-Phe-Ser-

*(where X is undetermined)

As determined by Edman Degradation, each subunit probably having the same above sequence.

2. TGF-Beta Derived from Placenta

The acid-ethanol extract of human placenta displayed activity that stimulated anchorage-dependent NRK cells to form colonies in soft agar. EGF markedly enhanced (150 fold) the activity of this placental TGF. As has been previously demonstrated for other TGFs, the activity of a partially purified placental preparation was destroyed by treatment with either trypsin or dithiothreitol.

Chromatography of the undialyzed crude residue from the combined acid-ethanol extractions of 11 placentas on a column in 1 M acetic acid gave two peaks of activity when assayed in the presence of EGF (pool A, apparent $M_r$ 5,000-9,000 and pool C, apparent $M_r$ less than 3500). No colony stimulating activity was detected when equivalent aliquots were assayed in the absence of EGF. Therefore, all subsequent soft agar assays were performed in the presence of 2 ng/ml EGF. None of the 3 pools competed with $^{125}$I-EGF for EGF membrane receptor sites on CCL-64 cells. The placenta-derived TGF of the present invention is therefore clearly a member of the TGF-beta family.

Pool A, which contained 47% of the recovered protein, had 17% of the recovered TGF-beta activity (see Table 4) while pool C, with only 3.3% of the protein, contained 18% of the recovered activity. Pool B did not give a valid assay for TGF activity because of the presence of a growth inhibitory substance. This inhibitor could be separated from the soft agar colony forming activity by further chromatography. As indicated in Table 4, 69% of the TGF activity found in the crude residue was present in the pool B fraction that eluted from the column. Pools B and C were therefore used for further purification.

Application of the protein from the gel filtration column to a cation-exchange column, and subsequent elution of the applied material with a linear sodium chloride gradient, gave a single peak of soft agar colony forming activity. Although 85-96% of the applied protein was recovered from the column, only 10-45% of the applied TGF activity was detected. Whether this loss of activity is due to specific loss of the TGF protein, to denaturation of the TGF, or to the separation of the TGF from an activator is, at this time, not clear.

Fractions were pooled and chromatographed on high pressure liquid chromatography (HPLC) column using an acetonitrile-0.1% TFA gradient. The TGF activity for both pools eluted from the column as a single peak at an acetonitrile concentration of 35%. Rechromatography of this material on a CN support equilibrated in n-propanol-0.1% TFA yielded a single peak of TGF activity at 35% n-propanol which corresponded to a strong absorbance peak. The homogeneity of the final preparation was indicated by a constant amino acid composition (Table 5) across the peak as well as by gel electrophoresis. The final degree of purification of placenta derived TGF-beta from the crude extract was 110,000-124,000 fold with a 1.1% recovery of activity in pool C and 4.8% in pool B. Only 64-72 pg/ml of placental TGF-beta was needed to obtain a half-maximal growth stimulatory response ($ED_{50}$) in the presence of 2 ng/ml of EGF.

The purity of the final TGF preparation was also demonstrated by $NaDodSO_4$-polyacrylamide gradient gel electrophoresis. In the absence of beta-mercaptoethanol, a single polypeptide band with an apparent molecular weight of 23,000-25,000 was observed for TGF from either pool B or pool C. Reduction of the protein with beta-mercaptoethanol produced a single band at approximately 13,000 molecular weight. When the gel was sliced into 0.5 cm strips and the unreduced protein eluted into 1 M acetic acid, all the TGF activity was found in the slice that corresponded to a molecular weight of 23,000-25,000, clearly indicating that the TGF activity corresponded to the only detectable protein band. Thus, the purified, placenta-derived transforming growth factor of the present invention is a protein of molecular weight 23,000-25,000 and is composed of two polypeptide chains of approximately 13,000 molecular weight held together by disulfide linkages. Whether these chains are identical or different remains to be determined. Although the protein contains 16 half-cystine residues, it is not yet known whether all of these residues are involved in disulfide linkage. However, the extreme stability of the TGFs to acid treatment and heat denaturation suggests the presence of a large number of such bonds.

(A) Examples of Placenta-Derived TGF-Beta Extraction, Purification, and Analysis

Soft Agar Assay: Soft agar colony forming activity was determined as described previously except that the cells were stained at the end of one week in assay and the number and size of the colonies were determined using image analysis system.

Extraction: Normal term human placentas were frozen on dry ice within 30 minutes after delivery and stored at -60°C until used. Placentas were extracted using a procedure previously described in Roberts, et al., Proc. Nat. Acad. Sci. U.S.A. 77:3494-3498 (1980), except that the homogenized tissue (600-1000g) was stirred in the acid-ethanol solution at room temperature for 2 to 3 hr prior to centrifugation. The resulting supernatant was adjusted to pH 3.0 and protein precipitated with ether and ethanol. The precipitate was collected by filtration and redissolved in 1 M acetic acid (1 ml/g of tissue). Insoluble material was removed by centrifugation, the supernatant lyophilized and the residue (27 mg per gram wet weight placenta) stored at -20°C.

Gel Filtration Chromatography: The lyophilized extract (239 g) from 11 placentas (8.8 kg) was redissolved in 1 M acetic acid (50 mg residue per ml) and applied in two separate portions (107 g and 132 g residue) to a column (35.6 x 90 cm) containing acrylamide gel (100-200 mesh, equilibrated and eluted (1.6L/hr) with 1 M acetic acid at room temperature. Fractions (800 ml) were collected and aliquots of the even numbered fractions were assayed for protein and for growth promoting activity in soft agar. The fractions containing TGF activity were combined into three separate pools (A-C) and lyophilized. Pool B (6 g residue per column) was redissolved in 1 M acetic acid (60 mg/ml) and applied to a column (10 x 91 cm) containing P-6 acrylamide gel equilibrated with 1 M acetic acid. The protein was eluted from the column with 1 M acetic acid (150 ml/hr), collecting 37 ml fractions. Aliquots of even numbered fractions were assayed for TGF activity. The fractions containing this activity were pooled and lyophilized.

Ion-Exchange Chromatography: Twenty-four percent of pool B from the P-6 column (2.1 g protein) and pool C from the P-30 column (1.9 g protein) were redissolved separately in 60 ml 0.01 M acetic acid. The pH was adjusted to 4.5 and the conductivity to 1.2-1.5 mS/cm. Each sample was then applied to a cation exchange column (CM-Trisacryl M, LKB, 5 x 10 cm) equilibrated in 0.05 M sodium acetate, pH 4.5 (buffer A). The column was eluted with 300 ml of buffer A (145 ml/hr) followed by a linear sodium chloride gradient to 0.70 M sodium chloride in buffer A at 0.8 mM/min. After 70 fractions (29 ml/fraction), the column was washed with 1 M sodium chloride, 0.05 M sodium acetate, pH 2.5 and then reequilibrated with buffer A. Aliquots from the even numbered fractions were removed for determination of protein and TGF activity. The peak of activity was combined for further analysis.

Reverse-Phase HPLC: The sample from the ion-exchange column was made 10% (v/v) in acetonitrile, 0.1% (v/v) in trifluoroacetic acid (TFA) and the pH adjusted to 2.0. It was then pumped onto an HPLC column (10 $\mu$m particle size, 0.78 x 30 cm) equilibrated in acetonitrile:water:TFA (10:90:0.1), pH 2. After washing the sample onto the column with 50 ml of the initial solvent, the column was eluted (1.2 ml/min) with a 60 min linear gradient from 25:75:0.1 to 45:55:0.1 acetonitrile:water:TFA, pH 2. After 75 fractions (1.2 ml/fraction) the column was stripped with acetonitrile:water:TFA (80:20:0.1), pH 2, collecting 2.4 ml fractions. Aliquots (5 $\mu$l) were removed for assay of TGF activity.

The peak of TGF activity from the HPLC column was combined, lyophilized, redissolved in n-propanol:water:TFA (30:70:0.1) , pH 2, and applied to a CN column (10 $\mu$m particle size, 0.38 x 30 cm) equilibrated with the sample solvent. The column was then eluted (1.1 ml/min) with a 153 min linear gradient from 30:70:0.1 to 45:55:0.1 n-propanol:water:TFA, pH 2. Forty-five fractions (2.2 ml/fraction) were collected and aliquots were removed for bioassay, amino acid analysis, and gel electrophoresis.

NaDodSO$_4$-Polyacrylamide Gel Electrophoresis: Samples were analyzed on 1.5 mm slab gels using either a polyacrylamide gradient of 15 to 28% or a 15% polyacrylamide gel and a discontinuous buffer system. Proteins were fixed with formaldehyde and stained using a silver staining technique. In some cases, samples were boiled with 5% beta-mercaptoethanol for 3 min prior to application to the gel.

Other Procedures: Total protein was determined either by the dye-binding method or fluorescamine assay using bovine serum albumin as standard or by amino acid analysis. Quantitative amino acid analyses were done with a modified amino acid analyzer equipped with a fluorometer for the detection of primary amines using o-phthalaldehyde reagent solution and a computing integrator. Lyophilized samples (7-40 picomoles) were hydrolyzed in 100 $\mu$L constant boiling HCl containing 0.1% liquid phenol at 150° for 2 hr in sealed, evacuated tubes. These modified conditions of shortened hydrolysis at elevated temperature yielded results for several test proteins which were identical to or better than those obtained after conventional acid hydrolysis (110°, 24-48 hr). Assays for EGF-competing activity were performed as previously described.

A summary of a general extraction procedure found to be preferable to previously used procedures, is given below.


Example of a Placenta-Derived TGF-Beta Extraction Protocol


1. Placentas are placed on dry ice immediately after delivery and are stored at -70°C or colder until used.
2. Approximately 24 hr before extraction, thaw placentas at -20°C.
3. Chop 1 kg of placenta into pieces and place into extraction solution (4 L solution/kg tissue).
Extraction solution:     3189 ml 95% ethanol
770 ml water
66 ml concentrated HCl
210 mg phenylmethylsulfonyl fluoride
12 mg pepstatin A
4. Mince in a blender to give a slurry.

5. Stir slurry at room temperature for approximately 2 1/2 hr (requires heavy-duty stirrer).

6. Centrifuge - 17,000 x g - 10 min; Discard pellet, save supernatant.

7. Adjust the supernatant to pH 3.0 with concentrated ammonium hydroxide.

8. Add 0.55 volume of 5.5 M NaCl.

9. Precipitate overnight at 4°C.

10. Centrifuge - 17,700 x g - 10 min. Discard pellet, save supernatant.

11. Concentrate supernatant to 1/5 volume or less. (We used a hollow fiber concentrator with a 5000 MW nominal cutoff membrane).

12. Add 2 volumes 5.5 M NaCl to concentrated supernatant.

13. Precipitate overnight at 4°C.

14. Centrifuge - 17,700 x g - 10 min. Discard supernatant. Save pellet for gel filtration chromatography and further purification.

Table 4. Purification of TGF-Beta from Human Placenta.

| Purification step | Protein[1] recovered (mg) | ED$_{50}$[2] (ng/ml) | Specific[3] activity (units/ug) | Total activity (units x 10$^3$) | Degree of purification (fold) | Recovery of activity (%) |
|---|---|---|---|---|---|---|
| 1. Crude Extract | 239,000 | 7,600 | 0.09 | 21,510 | 1.0 | 100 |
| 2. Acrylamide Gel #1 | | | | | | |
|     Pool A | 73,900 | 15,000 | 0.05 | 3,695 | 0.6 | 17 |
|     Pool B | 27,720 | - | - | - | - | - |
|     Pool C | 1,900 | 360 | 2.0 | 3,800 | 22 | 18 |
| 3. Acrylamide Gel #2 | | | | | | |
|     Pool B | 8,700 | 410 | 1.7 | 14,790 | 19 | 69 |
| 4. Ion-Exchange | | | | | | |
|     Pool B[4] | 140 | 62 | 11.5 | 1,610 | 128 | 31 |
|     Pool C | 46.3 | 85 | 8.4 | 390 | 93 | 1.8 |
| 5. HPLC-C$_{18}$ | | | | | | |
|     Pool B | 0.27 | 0.10 | 7,000 | 1,900 | 77,000 | 37 |
|     Pool C | 0.26 | 1.2 | 595 | 155 | 6,610 | 0.7 |
| 6. HPLC-CN | | | | | | |
|     Pool B | 0.025 | 0.072 | 9,920 | 248 | 110,000 | 4.8 |
|     Pool C | 0.022 | 0.064 | 11,160 | 245 | 124,000 | 1.1 |

[1] For steps 1 to 4, total protein was determined by the dye-binding procedure (15). For steps 5 and 6, total protein was based on amino acid analysis.

[2] ED$_{50}$ is defined as the concentration (ng/ml) of TGF-Beta required to give a response of 1 unit in the presence of EGF (1 unit of activity gives 50% maximal response, approximately 1000 colonies < 3000 um$^2$ per plate).

[3] Specific activity = $\dfrac{1 \text{ unit}}{\text{ED}_{50} \text{ (total ml per petri dish)}}$ x 1000

[4] Twenty-four percent of pool B from step 3 was used for further purification.

Table 4 summarizes the examples and results of the purification. The purified human placenta-derived TGF-beta was then analyzed for its amino acid composition (Table 5) and sequence (Table 6) (using Edman Degradation) with the following results.

Table 5.   Amino Acid Composition of Human Placenta TGF-Beta

| amino acid | residues/mol (mean + range) |
|---|---|
| Aspartic acid | 24 ± 2 |
| Threonine | 8 ± 1 |
| Serine | 17 ± 1 |
| Glutamic acid | 25 ± 1 |
| Proline | ND |
| Glycine | 17 ± 4 |
| Alanine | 18 ± 1 |
| Half-cystine † | 16 ± 2 |
| Valine | 15 ± 2 |
| Methionine † | 2 ± 1 |
| Isoleucine | 11 ± 1 |
| Leucine | 24 ± 2 |
| Tyrosine | 17 ± 1 |
| Phenylalanine | 8 ± 1 |
| Histidine | 7 ± 1 |
| Lysine | 19 ± 2 |
| Tryptophan | ND |
| Arginine | 11 ± 1 |

* Triplicate samples (15 picomoles each) were hydrolyzed in constant boiling HCl containing 0.1% liquid phenol at 150° for 2h in sealed, evacuated tubes.  Values are calculated on the basis of an apparent molecular weight of 25,000.
† Determined by performic acid oxidation and acid hydrolysis.
        ND means not determined.

Table 6. Partial Amino Acid Sequence for Each of the Two Human Placenta TGF-Beta Subunits*

5                                    10
Ala-Leu-Asp-Thr-Asn-Tyr-CMC-Phe-(Ser-Ser-)

15                              20
Thr-Glu-Lys-Asn-CMC-CMC-Val-X-Gln-Leu-

25                          29
Tyr-Ile-Asp-Phe-X-(Lys)-Asp-Leu-Gly-

*(where CMC is Half-cystine or cystline, determined as S-carboxymethylcysteine and x is undetermined)

(B) Functional Characteristics of Placenta-Derived TGF-Beta

The presence of TGFs in the crude acid-ethanol extract of human placenta that were able to compete with EGF for binding to membrane receptors (TGF-$\alpha$s) has recently been noted in Stromberg, et al., Biochem. Biophys. Res. Commun. 106:354-361 (1982). In the TGF of the present invention, a significant amount of TGF-$\alpha$-like activity was not detected. Although some soft agar colony forming activity was found in the crude residue in the absence of EGF, this activity did not compete with EGF in a receptor binding assay and it was stimulated 150 fold by the addition of 2 ng/ml of EGF, indicating that most, if not all, of the TGF present was of the type beta class. Also, as the placental TGF was purified, it became totally dependent on exogenous EGF for soft agar colony forming activity. Part of this difference may be explained by the fact that in Stromberg, et al. (1982) colonies of 6 cells or greater were considered to be significant while for the present invention colonies had to contain at least 60 cells in order to be counted.

Because placenta contains much blood, it is possible that the placental TGF-beta of the present invention (10 ng per gram of tissue) originated from the platelets. However, even assuming that the placenta was 100% blood and that platelets comprised 0.2% of this blood, platelet TGF would account for only 8% of the recovered placental TGF. Also, blood platelets contain another peptide, platelet derived growth factor (PDGF). However, placental TGF-beta is not PDGF, as clearly demonstrated by the amino acid composition of TGF-beta (Table 2), which is different from that reported for PDGF, and by the results from two different assays. In the first assay; placental TGF-beta did not have any chemotactic activity when tested under conditions where PDGF displayed strong chemotactic activity. Similarly, placental TGF-beta did not compete with PDGF in a radioreceptor assay.

**Claims**
**Claims for the following Contracting States : FR, DE, IT, NL, SE, CH, LI, GB, BE**

1. Beta-type transforming growth factor derivable from human placentas, in the form of a homogeneous protein causing half-maximal growth stimulation of NRK-fibroblast indicator cells in soft agar at a protein concentration of less than $5 \times 10^{-12}$ M, wherein said cells comprise colonies larger than 3000 $\mu m^2$ and wherein said growth stimulation is increased approximately 1000-fold by the presence of EGF or TGF-$\alpha$, said factor being obtainable by a process, which comprises the preparation of an acid-ethanol extract of said human placentas, wherein the undialysed crude extract from said acid-ethanol extraction is chromatographed on a gel filtration column in 1 M acetic acid, the protein from the gel filtration column is applied to a cation-exchange column and subsequently eluted with a linear sodium chloride gradient, the fractions are pooled and chromatographed on high pressure liquid chromatography (HPLC) column using an acetonitrile -0.1 % trifluoroacetic acid (TFA) gradient, and the peak of activity from the HPLC column is rechromatographed on a CN support equilibrated in n-propanol-0.1 % TFA.

2. Beta-type transforming growth factor derivable from human blood platelets, in the form of a homogeneous protein causing half-maximal growth stimulation of NRK-fibroblast indicator cells in soft agar at a protein concentration of less than $5 \times 10^{-12}$ M, wherein said cells comprise colonies larger than 3000 $\mu m^2$ and wherein said growth stimulation is increased approximately 1000-fold by the presence of EGF or TGF-$\alpha$, said factor being obtainable by a process, which comprises subjecting a solubilized platelet extract to a two-column procedure of sequential gel filtation on acrylamide gel in 1M acetic acid, wherein the first gel filtration is carried out in the absence of urea at a flow rate of 20 ml/h and the second gel filtration is carried out at a flow rate of 3 ml/h in the presence of 8 M ultra-pure urea, the latter being dissolved at pH 2 in 1 M acetic acid to preclude the formation of cyanate, which gel filtration is followed by dialysis of peak activity fractions against 1M acetic acid to remove urea.

3. A purified beta-type transforming growth factor according to claim 1 or claim 2, wherein said factor has a molecular weight of 25,000.

4. A purified beta-type transforming growth factor according to any of the claims 1 to 3, wherein said factor comprises two subunits, each of said subunits having a partial amino acid sequence as determined by Edman Degradation comprising the following amino acids: alanine, leucine, aspartic acid, threonine, asparagine, tyrosine, phenylalanine, and serine.

5. Use of a product according to any of claims 1 to 4 for the production of a medicine therapeutically effective in enhancement of wound healing and tissue repair, with strong growth promoting ability.

**Claims for the following Contracting State : AT**

1. A process for preparing beta-type transforming growth factor derivable from human blood platelets, in the form of a homogeneous protein causing half-maximal growth stimulation of NRK-fibroblast indicator cells in soft agar at a protein concentration of less than $5 \times 10^{-12}$ M, wherein said cells comprise colonies larger than 3000 $\mu m^2$ and wherein said growth stimulation is increased approximately 1000-fold by the presence of EGF or TGF-$\alpha$, which comprises subjecting a solubilized platelet extract to a two-column procedure of sequential gel filtation on acrylamide gel in 1M acetic acid, wherein the first gel filtration is carried out in the absence of urea at a flow rate of 20 ml/h and the second gel filtration is carried out at a flow rate of 3 ml/h in the presence of 8 M ultrapure urea, the latter being dissolved at pH 2 in 1 M acetic acid to preclude the formation of cyanate, which gel filtration is followed by dialysis of peak activity fractions against 1M acetic acid to remove urea.

2. A process for preparing beta-type transforming growth factor derivable from human placentas, in the form of a homogeneous protein causing half-maximal growth stimulation of NRK-fibroblast indicator cells in soft agar at a protein concentration of less than $5 \times 12^{-12}$ M, wherein said cells comprise colonies larger than 3000 $\mu m^2$ and wherein said growth stimulation is increased approximately 1000-fold by the presence of EGF or TGF-$\alpha$, wherein an acid-ethanol extract of said human placentas is prepared, the undialyzed crude extract from said acid-ethanol extraction is chromatographed on a gel filtration column in 1 M acetic acid, the protein from the gel filtration column is applied to a cation-exchange column and subsequently eluted with a linear sodium chloride gradient, the fractions are pooled and chromatographed on high pressure liquid chromatography (HPLC) column using an acetonitrile-0.1% trifluoroacetic acid (TFA) gradient, and the peak of activity from the HPLC column is rechromatographed on a CM support equilibrated in n-propanol-0.1% TFA.

3. The process according to claim 1 or 2, wherein said factor has a molecular weight of 25,000.

4. The process according to any of the claims 1 to 3, wherein said factor comprises two subunits, each of said subunits having a partial amino acid sequence as determined by Edman Degradation comprising the following amino acids: alanine, leucine, aspartic acid, threonine, asparagine, tyrosine, phenylalanine, and serine.

5. Use of a product obtainable by the process according to any of claims 1 to 4 for the production of a medicine therapeutically effective in enhancement of wound healing and tissue repair, with strong growth promoting ability.

**Patentansprüche**
**Patentansprüche für folgende Vertragsstaaten : FR, DE, IT, NL, SE, CH, LI, GB, BE**

1. Transformierender Wachstumsfaktor des $\beta$-Typs, der von menschlicher Plazenta herleitbar ist, in Form eines homogenen Proteins, das die Hälfte der maximalen Wachstumsstimulation von NRK-Fibroblasten-Indikatorzellen in Weichagar bei einer Proteinkonzentration von weniger als 5 x $10^{-12}$ molar verursacht, wobei diese Zellen Kolonien, die größer sind als 3000 $\mu m^2$, umfassen, und wobei die Wachstumsstimulation durch die Gegenwart von EGF oder TGF-$\alpha$ um etwa das 1000-fache erhöht wird, wobei dieser Faktor mit Hilfe eines Verfahrens erhältlich ist, welches umfaßt die Herstellung eines Säure-Ethanol-Extrakts der menschlichen Plazentas umfaßt, wobei der undialysierte Rohextrakt aus der Säure-Ethanol-Extraktion an einer Gelfiltrationskolonne in 1-molarer Essigsäure chromatographiert wird, das Protein aus der Gelfiltrationskolonne auf eine Kationenaustauscherkolonne aufgegeben und danach mit Hilfe eines linearen Natriumchlorid-Gradienten eluiert wird, die Fraktionen miteinander vereinigt und auf einer Hochdruck-Flüssigkeitschromatographie (HPLC)-Kolonne unter Anwendung eines Acetonitril-0,1 %-Trifluoressigsäure (TFA)-Gradienten chromatographiert werden und die Fraktion mit dem Aktivitätsmaximum aus der HPLC-Kolonne wieder der Chromatographie an einem CN-Träger, der in n-Propanol-0,1 % TFA äquilibriert wurde, unterworfen wird.

2. Transformierender Wachstumsfaktor des $\beta$-Typs, der von menschlichen Blutplättchen herleitbar ist, in Form eines homogenen Proteins, das die Hälfte der maximalen Wachstumsstimulation von NRK-Fibroblasten-Indikatorzellen in Weichagar bei einer Proteinkonzentration von weniger als 5 x $10^{-12}$ molar verursacht, wobei diese Zellen Kolonien, die größer sind als 3000 $\mu m^2$, umfassen, und wobei die Wachstumsstimulation durch die Gegenwart von EGF oder TGF-$\alpha$ um etwa das 1000-fache erhöht wird, wobei dieser Faktor mit Hilfe eines Verfahrens erhältlich ist, bei dem ein löslich gemachter Blutplättchenextrakt einem ,Zwei-Kolonnen-Verfahren durch aufeinanderfolgende Gelfiltration an Acrylamidgel in 1-molarer Essigsäure unterworfen wird, wobei die erste Gelfiltration in Abwesenheit von Harnstoff bei einer Fließrate von 20 ml/h und die zweite Gelfiltration bei einer Fließrate von 3 ml/h in Gegenwart von 8-molarem ultrareinem Harnstoff durchgeführt wird, wobei der letzere bei pH 2 in 1-molarer Essigsäure gelöst wurde, um die Bildung von Cyanat auszuschließen, und wobei auf die Gelfiltration die Dialyse der Fraktionen mit Aktivitätsmaxima gegen 1-molare Essigsäure zur Entfernung von Harnstoff folgt.

3. Gereinigter transformierender Wachstumsfaktor des $\beta$-Typs nach Anspruch 1 oder Anspruch 2, wobei der Faktor ein Molekulargewicht von 25 000 hat.

4. Gereinigter transformierender Wachstumsfaktor des $\beta$-Typs nach einem der Ansprüche 1 bis 3, wobei der Faktor zwei Untereinheiten umfaßt und jede dieser Untereinheiten eine durch Edman-Abbau bestimmte partielle Aminosäuresequenz aufweist, welche die folgenden Aminosäuren umfaßt : Alanin, Leucin, Asparaginsäure, Threonin, Asparagin, Tyrosin, Phenylalanin und Serin.

5. Verwendung eines Produkts nach einem der Ansprüche 1 bis 4 zur Herstellung eines Arzneimittels, das therapeutisch wirksam zur Verbesserung der Wundheilung und der Gewebewiederherstellung ist und das starke wachstumsfördernde Wirksamkeit hat.

**Patentansprüche für folgenden Vertragsstaat : AT**

1. Verfahren zur Herstellung von transformierendem Wachstumsfaktor des $\beta$-Typs, der von menschlichen Blutplättchen herleitbar ist, in Form eines homogenen Proteins, das die Hälfte der maximalen Wachstumsstimulation von NRK-Fibroblasten-Indikatorzellen in Weichagar bei einer Proteinkonzentration von weniger als 5 x $10^{-12}$ molar verursacht, wobei diese Zellen Kolonien, die größer sind als 3000 $\mu m^2$, umfassen, und wobei die Wachstumsstimulation durch die Gegenwart von EGF oder TGF-$\alpha$ um etwa das 1000-fache erhöht wird, das darin besteht, daß ein löslich gemachter Blutplättchenextrakt einem Zwei-Kolonnen-Verfahren durch aufeinanderfolgende Gelfiltration an Acrylamidgel in 1-molarer Essigsäure unterworfen wird, wobei die erste Gelfiltration in Abwesenheit von Harnstoff bei einer Fließrate von 20 ml/h und die zweite Gelfiltration bei einer Fließrate von 3 ml/h in Gegenwart von 8-molarem ultrareinem Harnstoff durchgeführt wird, wobei der letztere bei pH 2 in 1-molarer Essigsäure gelöst wurde, um die Bildung von Cyanat auszuschließen, und wobei auf die Gelfiltration die Dialyse der Fraktionen mit Aktivitätsmaxima gegen 1-molare Essigsäure zur Entfernung von Harnstoff folgt.

2. Verfahren zur Herstellung von transformierendem Wachstumsfaktor des $\beta$-Typs der von menschlicher Plazenta herleitbar ist, in Form eines homogenen Proteins, das die Hälfte der maximalen Wachstumsstimulation von NRK-Fibroblasten-Indikatorzellen in Weichagar bei einer Proteinkonzentration von weni-

14

ger als 5 x $10^{-12}$ molar verursacht, wobei diese Zellen Kolonien, die größer sind als 3000 $\mu m^2$, umfassen, und wobei die Wachstumsstimulation durch die Gegenwart von EGF oder TGF-$\alpha$ um etwa das 1000-fache erhöht wird, wobei ein Säure-Ethanol-Extrakt der menschlichen Plazentas hergestellt wird, der undialysierte Rohextrakt aus der Säure-Ethanol-Extraktion an einer Gelfiltrationskolonne in 1-molarer Essigsäure chromatographiert wird, das Protein aus der Gelfiltrationskolonne auf eine Kationenaustauscherkolonne aufgegeben und danach mit Hilfe eines linearen Natriumchlorid-Gradienten eluiert wird, die Fraktionen miteinander vereinigt und auf einer Hochdruck-Flüssigkeitschromatographie (HPLC)-Kolonne unter Anwendung eines Acetonitril-0,1 %-Trifluoressigsäure (TFA)-Gradienten chromatographiert werden und die Fraktion mit dem Aktivitätsmaximum aus der HPLC-Kolonne wieder der Chromatographie an einem CN-Träger, der in n-Propanol-0,1 % TFA äquilibriert wurde, unterworfen wird.

3. Verfahren nach Anspruch 1 oder 2, wobei dieser Faktor ein Molekulargewicht von 25 000 hat.

4. Verfahren nach einem der Ansprüche 1 bis 3, wobei der Faktor zwei Untereinheiten umfaßt und jede dieser Untereinheiten eine durch Edman-Abbau bestimmte partielle Aminosäuresequenz aufweist, welche die folgenden Aminosäuren umfaßt : Alanin, Leucin, Asparaginsäure, Threonin, Asparagin, Tyrosin, Phenylalanin und Serin.

5. Verwendung eines Produkts, das mit Hilfe eines Verfahrens nach einem der Ansprüche 1 bis 4 erhältlich ist, zur Herstellung eines Arzneimittels, das therapeutisch wirksam zur Verbesserung der Wundheilung und der Gewebewiederherstellung ist und das starke wachstumsfördernde Wirksamkeit hat.

**Revendications**
**Revendications pour les Etats contractants suivants : FR, DE, IT, NL, SE, CH, LI, GB, BE**

1. Facteur de croissance transformant du type béta, pouvant provenir de placentas humains, sous la forme d'une protéine homogène provoquant une stimulation de croissance semi-maximale de cellules indicatrices de fibroblastes NRK dans de l'agar mou, à une concentration protéinique inférieure à 5 x $10^{-12}$ M, lesdites cellules comprenant des colonies supérieures à 3000 $\mu m^2$ et ladite stimulation de croissance étant augmentée d'environ 1000 fois par suite de la présence d'EGF ou de TGF-$\alpha$, ledit facteur pouvant être obtenu par un procédé consistant en ce que l'on prépare un extrait acide-éthanolique desdits placentas humains, on chromatographie l'extrait brut non dialysé de ladite extraction acide-éthanolique sur une colonne de gel de filtration dans de l'acide acétique 1 M, on fait passer la protéine de la colonne de gel de filtration sur une colonne échangeuse d'ions et ensuite, on élue avec un gradient linéaire de chlorure de sodium, on réunit les fractions et l'on chromatographie sur une colonne de chromatographie liquide haute pression (HPLC) au moyen d'un gradient acétonitrile-0,1 % acide trifluoroacétique (TFA), et l'on rechromatographie le pic d'activité de la colonne HPLC sur un support CN équilibré dans du n-propanol-0,1 % TFA.

2. Facteur de croissance transformant du type béta, pouvant provenir de plaquettes de sang humain, sous la forme d'une protéine homogène provoquant une stimulation de croissance semi-maximale de cellules indicatrices de fibroblastes NRK dans de l'agar mou, à une concentration protéinique inférieure à 5 x $10^{-12}$ M, lesdites cellules comprenant des colonies supérieures à 3000 $\mu m^2$ et ladite stimulation de croissance étant augmentée d'environ 1000 fois par suite de la présence d'EGF ou de TGF-$\alpha$, ledit facteur pouvant être obtenu par un procédé qui consiste à soumettre un extrait de plaquette solubilisé à un traitement sur deux colonnes de filtration séquentielle sur gel, sur un gel acrylamide dans de l'acide acétique 1 M, dans lequel la première filtration sur gel est réalisée en l'absence d'urée, avec un débit de 20 ml/h, et la seconde filtration sur gel est réalisée avec un débit de 3 ml/h, en présence de 8 M d'urée ultra-pure, cette dernière étant dissoute à pH 2 dans de l'acide acétique 1 M pour éviter la formation de cyanate, ladite filtration sur gel étant suivie par une dialyse des fractions des pics d'activité contre de l'acide acétique 1 M pour éliminer l'urée.

3. Facteur de croissance transformant du type béta purifié, selon la revendication 1 ou 2, dans lequel ledit facteur a une masse moléculaire de 25 000.

4. Facteur de croissance transformant du type béta purifié, selon l'une quelconque des revendications 1 à

EP 0 128 849 B1

3, dans lequel ledit facteur comprend deux sous-ensembles, chacun desdits sousensembles ayant une séquence partielle d'aminoacides telle que déterminée par la Dégradation d'Edman, comprenant les aminoacides suivants : alanine, leucine, acide aspartique, thréonine, asparagine, tyrosine, phénylalanine et sérine.

5. Utilisation d'un produit selon l'une quelconque des revendications 1 à 4, pour la préparation d'un médicament qui est thérapeutiquement efficace pour favoriser la guérison des plaies et pour réparer des tissus, tout en ayant une forte action favorisant la croissance.

**Revendications pour l'Etat contractant suivant : AT**

1. Procédé pour la préparation de facteur de croissance transformant du type béta, pouvant provenir de plaquettes de sang humain, sous la forme d'une protéine homogène provoquant une stimulation de croissance semi-maximale de cellules indicatrices de fibroblastes NRK dans de l'agar mou, à une concentration protéinique inférieure à $5 \times 10^{-12}$ M, lesdites cellules comprenant des colonies supérieures à 3000 $\mu m^2$ et ladite stimulation de croissance étant augmentée d'environ 1000 fois par suite de la présence d'EGF ou de TGF-$\alpha$, procédé qui consiste à soumettre un extrait de plaquette solubilisé à un traitement sur deux colonnes de filtration séquentielle sur gel, sur un gel acrylamide dans de l'acide acétique 1 M, dans lequel la première filtration sur gel est réalisée en l'absence durée, avec un débit de 20 ml/h, et la seconde filtration sur gel est réalisée avec un débit de 3 ml/h, en présence de 8 M durée ultra-pure, cette dernière étant dissoute à pH 2 dans de l'acide acétique 1 M pour éviter la formation de cyanate, ladite filtration sur gel étant suivie par une dialyse des fractions des pics d'activité contre de l'acide acétique 1 M pour éliminer l'urée.

2. Procédé pour la préparation de facteur de croissance transformant du type béta, pouvant provenir de plaquettes de sang humain, sous la forme d'une protéine homogène provoquant une stimulation de croissance semi-maximale de cellules indicatrices de fibroblastes NRK dans de l'agar mou, à une concentration protéinique inférieure à $5 \times 10^{-12}$ M, lesdites cellules comprenant des colonies supérieures à 3000 $\mu m^2$ et ladite stimulation de croissance étant augmentée d'environ 1000 fois par suite de la présence d'EGF ou de TGF-$\alpha$, procédé dans lequel on prépare un extrait acide-éthanolique desdits placentas humains, on chromatographie l'extrait brut non dialysé de ladite extraction acide-éthanolique sur une colonne de gel de filtration dans de l'acide acétique 1 M, on fait passer la protéine de la colonne de filtration sur gel sur une colonne échangeuse d'ions et ensuite, on élue avec un gradient linéaire de chlorure de sodium, on réunit les fractions et l'on chromatographie sur une colonne de chromatographie liquide haute pression (HPLC) au moyen d'un gradient acétonitrile-0,1 % acide trifluoroacétique (TFA), et l'on rechromatographie le pic de la colonne HPLC sur un support CN équilibré dans du n-propanol-0,1 % TFA.

3. Procédé selon la revendicaton 1 ou 2, dans lequel ledit facteur a une masse moléculaire de 25 000.

4. Procédé selon l'une quelconque des revendications 1 à 3, dans lequel ledit facteur comprend deux sous-ensembles, chacun desdits sous-ensembles ayant une séquence partielle d'aminoacides telle que déterminée par la Dégradation d'Edman, comprenant les aminoacides suivants : alanine, leucine, acide aspartique, thréonine, asparagine, tyrosine, phénylalanine et sérine.

5. Utilisation d'un produit pouvant être obtenu par le procédé selon l'une quelconque des revendications 1 à 4, pour la préparation d'un médicament qui est thérapeutiquement efficace pour favoriser la guérison des plaies et pour réparer les tissus, tout en ayant une forte action favorisant la croissance.